# EUROPEAN PATENT APPLICATION

(11) **EP 4 299 054 A1**
(43) Date of publication of application: **03.01.2024**
(21) Application number: 23181588.7
(22) Date of filing: 26.06.2023
(51) Int. Cl.: A61K 9/20, A61K 31/7048

(54) **A TABLET COMPRISING EMPAGLIFLOZIN**

(30) Priority: 29.06.2022 TR 202210735
(71) Applicant: SANOVEL ILAC SANAYI VE TICARET A.S., Istanbul 34460 (TR)
(72) Inventor: GULER, Tolga, Istanbul (TR); ARMUT, Merve, Istanbul (TR); PEHLIVAN AKALIN, Nur, Istanbul (TR); SUNEL, Fatih, Istanbul (TR)
(74) Representative: Sevinç, Erkan

(57) **Abstract**

The present invention relates to a film coated tablet comprising empagliflozin or a pharmaceutically acceptable salt thereof and at least two diluents, wherein the tablet is obtained by direct compression. The process is simple, rapid, cost effective, time-saving and industrially convenient process and is created to eliminate the disadvantages of active ingredient.

## Description

### Field of the Invention

The present invention relates to a film coated tablet comprising empagliflozin or a pharmaceutically acceptable salt thereof and at least two diluents, wherein the tablet is obtained by direct compression. The process is simple, rapid, cost effective, time-saving and industrially convenient process and is created to eliminate the disadvantages of active ingredient.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2: Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose. In Type 2 diabetes, the pancreas makes insulin, but it either doesn't produce enough, or the insulin does not work properly. This diabetes occurs most often in people who are over 40 years old and overweighed. Type 2 diabetes may sometimes be controlled with a combination of diet, weight management, and exercise. However, treatment also may include oral glucose-lowering medications or insulin injections.

Empagliflozin is a known SGLT2 inhibitor that is described for the treatment or improvement in glycemic control in patients with type 2 diabetes mellitus. The chemical name of empagliflozin is 1 - chloro-4-(3-D-glucopyranos-1 -yl)- 2-[4-((S)-tetrahydrofuran-3-yloxy)-benzyl]-benzene and its chemical structure is shown in the Formula I.

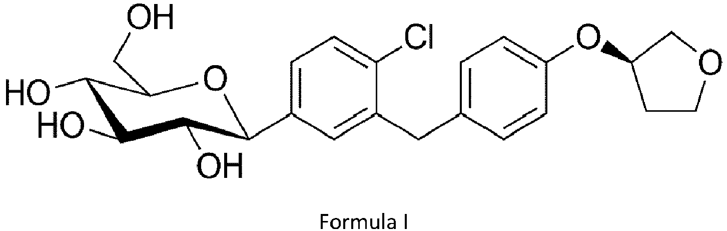

Empagliflozin is available on the market in the form of a free base and is sold under trade name Jardiance<^{®}>, as an oral tablet in 10 mg and 25 mg strengths. Further it is available on the market as a combination product with Metformin and a combination product with Linagliptin.

Active ingredients have some disadvantages in the formulation and process. The main problem encountered when preparing formulations comprising empagliflozin is low solubility, leading to difficulties with disintegration and dissolution profile.

Patent CN102387783A improves the dissolution rate of empagliflozin by controlling the particle size distribution of the raw material and the particle size of the drug particles, but the preparation method is not effective, and the loss of active agent is large, and it is easy to agglomerate and agglomerate after the micro powder, which virtually reduces the production efficiency, so cause stability problems.

Patent CN104623684A prepares mannitol and empagliflozin into a solid dispersion through solid dispersion technology to improve the dissolution rate.

In the prior art, several patents have been done for the dissolution profile. However, since empagliflozin is an active ingredient that has stability problems and is used in small amounts in formulation, focusing only on its dissolution profile will not yield successful results. Therefore, formulation and process steps have gained importance.

There still remains a need in the art to provide an improved a film coated tablet comprising empagliflozin having excellent pharmacomechanic properties, long-term stability and high solubility which is also obtained by using an effective process. In the present invention, the film coated tablet is created to overcome the above problems and provides additional advantages to the relevant field of art. Other advantages and embodiments of the present invention will be clarified in the following description.

### Detailed Description of the Invention

The present invention relates to a film coated tablet comprising empagliflozin or a pharmaceutically acceptable salt thereof, wherein the film coated tablet is obtained by direct compression. So, this way eliminates the problems stability of active agent and provides additional advantages to the relevant field of art.

The main object of the present invention is to provide a desired stability of the film coated tablet comprising empagliflozin or a pharmaceutically acceptable salt thereof.

Another object of the present invention is to provide a film coated tablet comprising empagliflozin or a pharmaceutically acceptable salt thereof with excellent physicochemical properties, such as compressibility, flowability, homogeneity, and content uniformity which overcomes the above-described problems in the prior art and have additive advantages over them.

Another object of the present invention is to provide a process for preparing a film coated tablet comprising empagliflozin or a pharmaceutically acceptable salt thereof. The process is direct compression, it is a simple, rapid, cost effective, time-saving, and industrially convenient method.

Empagliflozin or a pharmaceutically acceptable salt thereof is used small proportion that can lead to considerable problems during the manufacture of the composition with regard to the uniformity of the content of active agent and flowability in the individual composition units.

Flowability is an important parameter in tablet formulation. It can be a problem especially in formulations containing a small amount of active substance. In a formulation, the problem with flowability negatively affects the content uniformity, and the problem with the content uniformity negatively affects the dissolution profile. We have found in this invention that a particularly at least two diluents overcome these problems.

Empagliflozin is unstable and prone to impurities. In addition to affecting the efficacy of drugs, impurities may increase the risk of toxic and side effects. In this invention, to eliminate this problem, a process which is free of solvent is preferred in terms of the desired stability. In using process, preferably direct compression, empagliflozin and other excipients are not exposed to moisture and solvents. Thanks to this way eliminates the problems stability of empagliflozin and provides additional advantages to the relevant field of art.

According to an embodiment of the present invention, a film coated tablet comprising empagliflozin or a pharmaceutically acceptable salt thereof and at least two diluents, wherein the tablet is obtained by direct compression. The film coated tablet is obtained which provides the desired stability and good flowability of the powder/granules.

Suitable diluents are selected from the group comprising microcrystalline cellulose, anhydrous lactose, lactose, corn starch, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

Used at least two diluents eliminates the disadvantages of both active agents and provides an effective tablet formulation.

According to an embodiment of the present invention, the diluents are microcrystalline cellulose and anhydrous lactose. The diluent provides flowability and compressibility of the powder mixture. Also, these diluents help to provide the desired stability.

According to an embodiment of the present invention, the amount of diluents is 10.0% to 85.0% by weight in the total composition. Preferably, the amount of diluents is 30.0% to 80.0% by weight in the total composition.

According to one embodiment of the present invention, the film coated tablet further comprises at least one glidant and at least one lubricant.

Suitable lubricant is selected from the group comprising talc, sodium stearyl fumarate, magnesium stearate, potassium stearate, stearic acid, sodium chloride, sodium benzoate, sodium acetate, sodium oleate, polyethylene glycols or mixtures thereof.

According to one embodiment of the present invention, the lubricant is talc or sodium stearyl fumarate or magnesium stearate or mixtures thereof. These excipients provide the flowability of the powder/granules.

According to one embodiment of the present invention, the amount of lubricant is between 0.1% and 10.0% by weight in the total formulation. Preferably, the amount of lubricant is 0.1% to 5.0% by weight in the total composition.

Suitable glidants are selected from the group comprising anhydrous colloidal silicon dioxide, hydrophilic polymers, silicon dioxide or mixtures thereof.

According to one embodiment of the present invention, the glidant is anhydrous colloidal silicon dioxide.

According to one embodiment of the present invention, the amount of glidant is between 0.1% and 10.0% by weight in the total formulation. Preferably, the amount of glidant is 0.1% to 5.0% by weight in the total composition.

The term "Dx" as used herein means that x% of the particles in a composition (based on volume) have a diameter of or below a specified d value. D (0.9)" or "d90" means that the size at which %90 by volume of the particles are finer. The volume mean particle size of the compound of the Empagliflozin is determined using Malvern Mastersizer 2000 laser diffraction particle size analyzer.

According to one embodiment of this invention, Empagliflozin or a pharmaceutically acceptable salt thereof has a d (0.9) particle size between 100 µm and 350 µm. These particle size helps to provide the desired content uniformity.

According to this embodiment, Empagliflozin or a pharmaceutically acceptable salt thereof has a d (0.9) particle size between 100 µm to 180 µm, between 180 µm to 250 µm, between 250 µm to 300 µm, between 300 µm to 350 µm.

According to one embodiment of the present invention, the tablet comprises;
- 0.5% and 25.0% by weight of empagliflozin,
- 10.0% and 85.0% by weight of at least two diluents,
- 0.1% and 10.0% by weight of lubricant,
- 0.1% and 10.0% by weight of glidant of the total formulation.

According to one embodiment of the present invention, the tablet comprises;
- 0.5% and 25.0% by weight of empagliflozin,
- 10.0% and 85.0% by weight of microcrystalline cellulose and lactose anhydrous
- 0.1% and 10.0% by weight of lubricant,
- 0.1% and 10.0% by weight of glidant of the total formulation.

According to one embodiment of the present invention, the tablet comprises;
- Empagliflozin
- Microcrystalline cellulose
- Lactose anhydrous
- Anhydrous colloidal silicon dioxide
- Magnesium stearate or sodium stearyl fumarate

According to one embodiment of the present invention, a process for the preparation of the film coated tablet comprises the following steps:
a) Mixing empagliflozin, microcrystalline cellulose, lactose anhydrous and anhydrous colloidal silicon dioxide,
b) Sieving the mixture through 630 µm and then mixing,
c) Adding magnesium stearate or sodium stearyl fumarate, and then mixing,
d) Compressing the mixture into tablets.
e) Coating the tablets with film coating.

### Example 1:

| **Ingredients** | **Amount (% by weight of the total formulation)** |
|---|---|
| Empagliflozin | 0.5 -25.0 |
| Microcrystalline cellulose | 5.0 - 70.0 |
| Lactose anhydrous | 5.0 - 70.0 |
| Anhydrous colloidal silicon dioxide | 0.1 -10.0 |
| Magnesium stearate or sodium stearyl fumarate | 0.1 -10.0 |
| **TOTAL** | **100** |

### Example 2:

| **Ingredients** | **Amount (% by weight of the total formulation)** |
|---|---|
| Empagliflozin | 3.0 -20.0 |
| Microcrystalline cellulose | 20.0 - 60.0 |
| Lactose anhydrous | 10.0 - 60.0 |
| Anhydrous colloidal silicon dioxide | 0.1 -6.0 |
| Magnesium stearate or sodium stearyl fumarate | 0.1 -6.0 |
| **TOTAL** | **100** |

A process for example 1 or 2;
a) Mixing empagliflozin, microcrystalline cellulose, lactose anhydrous and anhydrous colloidal silicon dioxide,
b) Sieving the mixture through 630 µm and then mixing,
c) Adding magnesium stearate or sodium stearyl fumarate, and then mixing,
d) Compressing the mixture into tablets.
e) Coating the tablets with film coating.

## Claims

1. A film coated tablet comprising empagliflozin or a pharmaceutically acceptable salt thereof and at least two diluents, wherein the tablet is obtained by direct compression.

2. The film coated tablet according to claim 1, wherein diluents are selected from the group comprising microcrystalline cellulose, anhydrous lactose, lactose, corn starch, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

3. The film coated tablet according to claim 1, wherein the diluents are microcrystalline cellulose and anhydrous lactose.

4. The film coated tablet according to claim 1, wherein the amount of diluents is 10.0% to 85.0% by weight in the total composition.

5. The film coated tablet according to claim 1, wherein the film coated tablet further comprises at least one glidant and at least one lubricant.

6. The film coated tablet according to claim 5, wherein lubricant is selected from the group comprising talc, sodium stearyl fumarate, magnesium stearate, potassium stearate, stearic acid, sodium chloride, sodium benzoate, sodium acetate, sodium oleate, polyethylene glycols or mixtures thereof.

7. The film coated tablet according to claim 5, wherein the lubricant is talc or sodium stearyl fumarate or magnesium stearate or mixtures thereof.

8. The film coated tablet according to claim 5, wherein glidants are selected from the group comprising anhydrous colloidal silicon dioxide, hydrophilic polymers, silicon dioxide or mixtures thereof.

9. The film coated tablet according to claim 5, wherein the glidant is anhydrous colloidal silicon dioxide.

10. The film coated tablet according to claim 1, wherein empagliflozin or a pharmaceutically acceptable salt thereof has a d (0.9) particle size between 100 µm and 350 µm.

11. The film coated tablet according to claim 1, wherein the tablet comprising;
- 0.5% and 25.0% by weight of empagliflozin,
- 10.0% and 85.0% by weight of at least two diluents,
- 0.1% and 10.0% by weight of lubricant,
- 0.1% and 10.0% by weight of glidant of the total formulation.

12. The film coated tablet according to claim 1, wherein the tablet comprising;
- 0.5% and 25.0% by weight of empagliflozin,
- 10.0% and 85.0% by weight of microcrystalline cellulose and lactose anhydrous
- 0.1% and 10.0% by weight of lubricant,
- 0.1% and 10.0% by weight of glidant of the total formulation.

13. The film coated tablet according to claim 1, wherein the film coated tablet comprising;
- Empagliflozin
- Microcrystalline cellulose
- Lactose anhydrous
- Anhydrous colloidal silicon dioxide
- Magnesium stearate or sodium stearyl fumarate

14. A process for the preparation of the film coated tablet comprises the following steps:
a) Mixing empagliflozin, microcrystalline cellulose, lactose anhydrous and anhydrous colloidal silicon dioxide,
b) Sieving the mixture through 630 µm and then mixing,
c) Adding magnesium stearate or sodium stearyl fumarate, and then mixing,
d) Compressing the mixture into tablets.
e) Coating the tablets with film coating.
